# EUROPÄISCHE PATENTSCHRIFT

(11) **EP 0 459 178 B1**
(45) Veröffentlichungstag und Bekanntmachung des Hinweises auf die Patenterteilung: **05.01.1994**
(21) Anmeldenummer: 91107277.5
(22) Anmeldetag: 04.05.1991
(51) Int. Cl.: A61F 13/15

(54) **Windel mit Dichtungsklappen**
Diaper with sealing cuffs
Couche avec barrières d'étanchéité

(30) Priorität: 25.05.1990 DE 4016864
(43) Veröffentlichungstag der Anmeldung: 04.12.1991
(73) Patentinhaber: VP - SCHICKEDANZ AG, D-90022 Nürnberg (DE)
(72) Erfinder: Petranyi, Pal, Dr., W-8500 Nürnberg 90 (DE); Lang, Hildegard, W-8500 Nürnberg 80 (DE)
(74) Vertreter: Rau, Manfred, Dr. Dipl.-Ing.

(56) Entgegenhaltungen:
- EP-A- 0 219 326
- GB-A- 2 161 059
- US-A- 4 846 825

## Beschreibung

Die Erfindung betrifft eine zum einmaligen Gebrauch bestimmte Windel, beispielsweise eine Kinderwindel oder auch eine Erwachsenenwindel, die in bekannter Weise mit einer flüssigkeitsdurchlässigen oberen Abdeckung, einer flüssigkeitsdichten unteren Abdeckung und einem zwischen den Abdeckungen angeordneten Saugkissen ausgestattet ist. Die flüssigkeitsdurchlässige obere Abdeckung besteht üblicherweise aus einem leichtgewichtigen Vliesstoff; die flüssigkeitsdichte untere Abdeckung, die sogenannte Wäscheschutzfolie besteht beispielsweise aus einer 25 µ starken Polyethylenfolie. Die beiden erwähnten Abdeckungen überragen das Saugkissen allseits, und sie sind unter Bildung eines Randlappens miteinander verklebt. Die Windel ist ferner mit zwei leistenartigen Dichtungsstreifen ausgestattet, die mit Abstand voneinander angeordnet sind, in Windellängsrichtung verlaufen, bei Gebrauch etwa lotrecht zur Oberfläche des Saugkissens stehen und zumindest einen Teil des Saugkissens zwischen sich begrenzen. Dabei sind sowohl in den Randlappen wie auch in den Dichtungslappen elastische Bänder oder Fäden angeordnet, die derart unter Spannung stehen, daß sie in der Gebrauchslage der Windel die sie umgebenden Teile leicht kräuseln.

Windeln der vorbeschriebenen Art sind grundsätzlich bekannt; sie sind beispielsweise in der EP-OS 219 326 beschrieben. Die erwähnten Dichtungslappen werden in der englischsprachigen Literatur, so auch in der europäischen Offenlegungsschrift 219 326 als "Cuffs" bezeichnet. Sie haben den Zweck, die Windel zur Seite hin zusätzlich abzudichten und dadurch den Tragekomfort und die Sicherheit zu erhöhen.

Die vorbekannten Windeln mit zusätzlichem längsverlaufenden Dichtungsstreifen (Cuffs) sind jedoch sowohl bei der Herstellung wie auch beim Gebrauch problematisch. Bei der Herstellung vieler Ausführungsformen ist es erforderlich, den zusätzlichen Dichtungsstreifen gesondert bereitzustellen und ihn, gegebenenfalls unter mechanischer Anspannung des im Dichtungsstreifen vorhandenen elastischen Bandes, in die Windel einzukleben. Um dies durchzuführen, sind verhältnismäßig aufwendige Maschinen erforderlich, wobei zudem der Nachteil auftritt, daß durch das Einbringen der zusätzlichen Dichtungsbänder und das damit verbundene Abbinden zusätzlicher Klebstoffnähte die Produktionsgeschwindigkeit begrenzt wird. Beim Gebrauch vieler Ausführungsformen derartiger Windeln ist es oft nachteilig, daß die zusätzlichen Dichtungsbänder mitunter Druckstellen bewirken, die das Tragen solcher Windeln weniger angenehm machen. Es rührt dies daher, daß die Randbereiche der Windeln zwangsläufig aus mehreren übereinander angeordneten Folienschichten bestehen, zu denen nun ja noch die zusätzlichen Dichtungsstreifen hinzukommen, wodurch sich leicht Falten bilden können, die die erwähnten Druckstellen verursachen.

Der Erfindung liegt die Aufgabe zugrunde, die vorbekannten Windeln mit zusätzlichen Dichtungsstreifen weiter zu entwickeln, ihre Herstellung zu vereinfachen sowie insbesondere den Tragekomfort und die seitliche Abdichtung zu verbessern.

Zur Lösung dieser Aufgabe wird vorgeschlagen, daß das Saugkissen über seine gesamte Länge durch zwei parallele und mit Abstand voneinander angeordnete Spalte unterbrochen ist, wodurch das Saugkissen in folgende Felder aufgeteilt wird, nämlich ein breites Mittelfeld und zwei schmale Seitenfelder. Im Bereich der Spalte sind die flüssigkeitsdurchlässige obere Abdeckung und die flüssigkeitsdichte untere Abdeckung gemeinsam unter Bildung der Dichtungslappen schleifenförmig hochgezogen. Die untere Abdeckung ist sodann in den Innenfeldern der schleifenförmigen Dichtungslappen zu deren Stabilisation miteinander verklebt.

Durch eine derartige Anordnung werden mehrere Vorteile erzielt: Zum einen wird durch die Aufspaltung des Saugkissens erreicht, daß sich beidseits der zusätzlichen Dichtungslappen etwa gleich hohe Polster befinden, die das Auftreten von Druckstellen verhindern oder wenigstens mindern. Zum anderen wird dadurch, daß die zusätzlichen Dichtungslappen nicht in einem gesonderten Arbeitsgang gebildet und dann in die Windel eingeklebt werden müssen, die Herstellung vereinfacht.

Die erwähnte Verklebung der Dichtungslappen in den Innenfeldern der Schleifen kann dabei ganzflächig erfolgen oder auch nur in Form einer schmalen Klebstoffspur oder einer Folge von Klebepunkten im unteren Bereich der Schlaufe.

Die zusätzlichen Dichtungslappen sollen bei Gebrauch etwa oder nahezu lotrecht von der Oberfläche der Windel abstehen. Es wird dies erreicht, indem in die Oberkante der Dichtungslappen entsprechende elastische Bänder eingearbeitet werden. Die beiden Enden der Dichtungslappen werden aber vorzugsweise flach gelegt und mit der Oberfläche der oberen Abdeckung verklebt.

Bei der Herstellung wird die Windel in bekannter Weise zusammengefaltet, wobei dann naturgemäß auch die gesamte Länge der beiden Dichtungslappen flach auf die Windeloberfläche gedrückt wird. Beim Auffalten der Windel werden aber die elastischen Bänder, die sich in den Dichtungslappen befinden, angespannt und dadurch die nicht mit der Oberfläche verklebten Abschnitte, also im wesentlichen die mittleren Gebrauchsabschnitte, aufgerichtet.

Die Erfindung wird im folgenden anhand der beigefügten Zeichnung näher erläutert. Es stellen dar:
- Fig. 1: eine perspektivische Ansicht einer erfindungsgemäßen Windel im halb aufgerichteten Zustand;
- Fig. 2: einen Querschnitt durch die Windel entlang der Linie II-II.

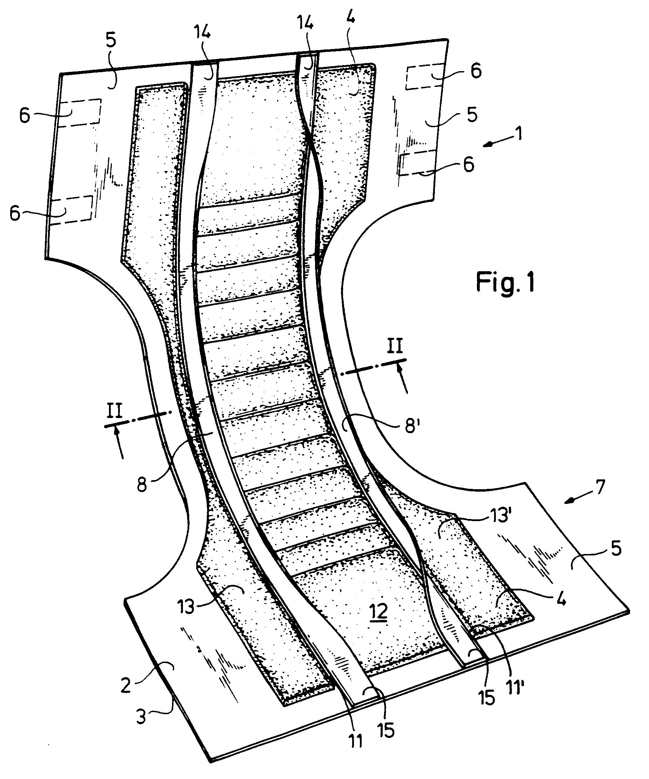

Die in Fig. 1 dargestellte Windel ist als Ganzes mit 1 bezeichnet. Sie besteht im wesentlichen aus einer flüssigkeitsdurchlässigen oberen Abdeckung 2, also beispielsweise einer Vliesstoffschicht, einer flüssigkeitsdichten unteren Abdeckung 3, also beispielsweise einer dünnen Polyethylenfolie, sowie einem zwischen den Abdeckungen angeordneten Saugkissen 4. Das Saugkissen besteht in bekannter Weise aus einer luftgelegten Ansammlung von Zellstoff-Flocken, denen gegebenenfalls noch weitere Stoffe zugemischt sein können, wie beispielsweise Quellstoffe, pH-regulierende Pufferstoffe, Desinfektionsmittel, Duftstoffe und dergleichen.

Die beiden Abdeckungen 2 und 3 überragen das Saugkissen 4 allseits, und sie sind unter Bildung eines Randlappens 5 miteinander verklebt.

Die Windel ist des weiteren noch in bekannter Weise mit Verschlußklebestreifen 6 ausgerüstet, die im verpackungsfertigen Zustand nach hinten um den Randstreifen herumgeklappt und dort provisorisch festgeklebt sind. Bei Gebrauch wird das herumgeklappte Ende des Verschlußstreifens nach außen gebogen, die Windel angelegt und das freie Ende der Verschlußstreifen an dem gegenüberliegenden Windelteil 7 unter Bildung einer Hose festgeklebt.

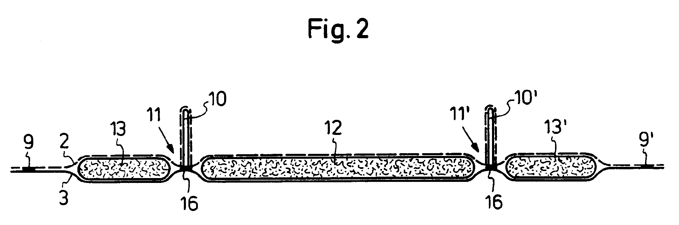

Fig. 1 läßt erkennen, daß die Windel ferner zwei leistenartige zusätzliche Dichtungsstreifen 8 und 8' aufweist, die mit Abstand voneinander angeordnet sind, die in Windellängsrichtung verlaufen und die bei Gebrauch etwa lotrecht zur Oberfläche des Saugkissens stehen und die zumindest einen Teil des Saugkissens zwischen sich begrenzen. Dabei sind sowohl in den Randlappen 5 elastische Bänder oder Fäden 9, 9' wie auch in den Dichtungsstreifen 8, 8' elastische Bänder oder Fäden 10, 10' angeordnet, wie dies aus Fig. 2 zu ersehen ist.

Wesentlich für die erfindungsgemäße Windel ist nun, daß das Saugkissen 4 über seine gesamte Länge durch zwei parallele und mit Abstand voneinander angeordnete Spalte 11, 11' unterbrochen ist, durch die das Saugkissen in folgende Felder aufgeteilt wird: ein breites Mittelfeld 12 und zwei schmale Seitenfelder 13 und 13'. Im Bereich dieser Spalte 11 und 11' sind nun beide Abdeckungen, also sowohl die flüssigkeitsdurchlässige obere Abdeckung 2 wie auch die flüssigkeitsdichte untere Abdeckung 3 gemeinsam unter Bildung der Dichtungsstreifen 8 und 8' schlaufenförmig hochgezogen. Um die Dichtungsstreifen zu stabilisieren, ist die untere Abdeckung 3 in den Innenfeldern der schlaufenförmigen Dichtungsstreifen 8 und 8' miteinander verklebt.

Die auf diese Weise gebildeten Dichtungsstreifen sind folglich beidseitig von relativ dicken und weichen Polstern umgeben. Auch ist deren Herstellung einfach und kann ohne Schwierigkeiten bei der laufenden Windelfertigung geschehen, ohne daß in einem gesonderten Arbeitsgang vorher bereitgestellte Dichtungsstreifen in die Windel eingebracht und eingeklebt werden müßten.

Fig. 2 zeigt die Anordnung besonders anschaulich im Querschnitt. Gleiche Teile sind dabei mit gleichen Bezugszeichen versehen.

Fig 1 zeigt des weiteren, daß die Dichtungsstreifen 8, 8' an ihren beiden Enden 14 und 15 flachgelegt und mit der Oberfläche der oberen Abdeckung verklebt sind.

Wie bereits gesagt, müssen die Innenfelder der schlaufenförmigen Dichtungsstreifen 8 und 8' miteinander verbunden, beispielsweise verklebt sein, um die Dichtungsstreifen zu stabilisieren. Diese Verklebung kann ganzflächig sein oder auch nur durch eine schmale Klebstoffspur oder eine Folge von Klebstoffpunkten bewirkt werden. Eine schmale Klebstoffspur ist im Ausführungsbeispiel der Fig. 2 angenommen und bei 16 dargestellt.

### Bezugszeichenliste

- 1 =: Windel
- 2 =: obere Abdeckung
- 3 =: untere Abdeckung
- 4 =: Saugkissen
- 5 =: Randlappen
- 6 =: Verschlußklebestreifen
- 7 =: Windelteil
- 8, 8' =: Dichtungsstreifen
- 9, 9' =: elastische Bänder
- 10, 10' =: elastische Bänder
- 11, 11' =: Spalte
- 12 =: Mittelfeld
- 13, 13' =: Seitenfelder
- 14 =: Enden der Dichtungsstreifen
- 15 =: Enden der Dichtungsstreifen
- 16 =: Klebstoffspur

## Patentansprüche

1. Windel mit
- einer flüssigkeitsdurchlässigen oberen Abdeckung (2)
- einer flüssigkeitsdichten unteren Abdeckung (3)
- einem zwischen den Abdeckungen angeordneten Saugkissen (4)
wobei die Abdeckungen (2,3) das Saugkissen (4) allseits überragen und unter Bildung eines Randlappens (5) miteinander verklebt sind;
- ferner mit zwei leistenartigen Dichtungsstreifen (8, 8'), die
-- mit Abstand voneinander angeordnet sind,
-- in Windellängsrichtung verlaufen,
-- bei Gebrauch etwa lotrecht zur Oberfläche des Saugkissens stehen und
-- zumindest einen Teil des Saugkissens zwischen sich begrenzen,
wobei sowohl in den Randlappen (5) wie auch in den Dichtungsstreifen (8, 8') elastische Bänder (9, 9', 10, 10') angeordnet sind,
gekennzeichnet durch folgende Merkmale:
- das Saugkissen (4) ist über seine gesamte Länge durch zwei parallele und mit Abstand voneinander angeordnete Spalte (11, 11') unterbrochen, durch die das Saugkissen in folgende Felder aufgeteilt wird:
-- ein breites Mittelfeld (12),
-- zwei schmale Seitenfelder (13, 13');
- im Bereich der Spalte (11, 11') sind
-- die flüssigkeitsdurchlässige obere Abdeckung (2) und
-- die flüssigkeitsdichte untere Abdeckung (3)
gemeinsam unter Bildung der Dichtungsstreifen (8, 8') schlaufenförmig hochgezogen;
- die untere Abdeckung (3) ist in den Innenfeldern der schlaufenförmigen Dichtungsstreifen (8, 8') zu deren Stabilisation miteinander verklebt.

2. Windel nach Anspruch 1,
dadurch gekennzeichnet,
daß die Dichtungsstreifen (8, 8') an ihren beiden Enden (14, 15) flachgelegt und mit der Oberfläche der oberen Abdeckung (2) verklebt sind.

3. Windel nach Anspruch 1 oder 2,
dadurch gekennzeichnet,
daß die untere Abdeckung (3) in den Innenfeldern der schlaufenförmigen Dichtungsstreifen (8, 8') ganzflächig verklebt sind.

4. Windel nach Anspruch 1 oder 2,
dadurch gekennzeichnet,
daß die untere Abdeckung (3) in den Innenfeldern der schlaufenförmigen Dichtungslappen (8, 8') durch eine schmale Klebstoffspur (16) oder eine Folge von Klebstoffpunkten verklebt ist, die sich im unteren Bereich der Dichtungslappen befinden.

## Claims

1. Diaper with
- a liquid-permeable upper covering (2)
- a liquid-proof lower covering (3)
- an absorbent pad (4) arranged between the coverings,
the coverings (2, 3) overlapping the absorbent pad (4) all around and being glued together thus forming a marginal flap (5);
- further with two strip-like sealing cuffs (8, 8') which
-- are spaced apart
-- extend in the longitudinal direction of the diaper
-- are about perpendicular to the surface of the absorbent pad during use and
-- define at least a part of the absorbent pad between them,
elastic tapes (9, 9', 10, 10') being arranged in the marginal flaps (5) as well as in the sealing cuffs (8, 8'), characterized by the following features:
- over its entire length the absorbent pad (4) is interspersed by two parallel gaps (11, 11') spaced apart dividing the absorbent pad into the following sections:
-- a wide central section (12),
-- two narrow lateral sections (13, 13');
- in the vicinity of the gap (11, 11')
-- the liquid-permeable upper covering (2) and
-- the liquid-proof lower covering (13)
are looped upwards to form the sealing cuffs (8, 8');
- the lower covering (3) is glued together at the inside portions of the loop-shaped sealing cuffs (8, 8') for their stabilization.

2. Diaper according to claim 1,
characterized in that
the sealing cuffs (8, 8') are laid flat at both their ends (14, 15) and are glued together with the surface of the upper covering (2).

3. Diaper according to claim 1 or 2,
characterized in that
the lower covering (3) is entirely glued together at the inside portions of the loop-shaped sealing cuffs (8, 8').

4. Diaper according to claim 1 or 2,
characterized in that
at the inside portions of the loop-shaped sealing cuffs (8, 8') the lower covering (3) is glued together by a narrow track of glue (16) or by a series of dots of glue located in the lower portion of the sealing cuffs.

## Revendications

1. Couche avec
- un revêtement supérieur (2) perméable aux liquides
- un revêtement inférieur (3) étanche aux liquides
- un coussin absorbant (4) arrangé entre les revêtements,
les revêtements (2, 3) s'étendant au-delà du coussin absorbant (4) tout autour et étant collés l'un à l'autre en formant une patte de bord (5);
- en plus, avec deux barrières d'étanchéité (8, 8') en forme de nervures, qui
-- sont entre-distantes
-- s'étendent dans la direction longitudinale de la couche
-- sont à-peu-près perpendiculaires à la surface du coussin absorbant pendant l'usage et
-- limitent entre eux au moins une partie du coussin absorbant,
des rubans élastiques (9, 9', 10, 10') étant arrangés dans les pattes de bord (5) si bien que dans les barrières d'étanchéité (8, 8'), caractérisée par les caractéristiques suivantes:
- le coussin absorbant (4) est interrompu en longueur totale par deux fentes (11, 11') parallèles et entre-distantes, par lesquelles le coussin absorbant est partagé dans les parties suivantes:
-- une large partie centrale (12),
-- deux parties latérales (13, 13') étroites;
- dans le domaine de la fente (11, 11')
-- le revêtement supérieur (2) perméable aux liquides et
-- le revêtement inférieur (3) étanche aux liquides sont en commun repliés en boucle formant des barrières d'étanchéité (8, 8');
- le revêtement inférieur (3) est contrecollé dans les zones intérieures des barrières d'étanchéité (8, 8') en boucle pour leur stabilisation.

2. Couche selon la revendication 1,
caractérisée en ce que
les barrières d'étanchéité (8, 8') sont mises à plat à leurs deux extrémités (14, 15) et collées avec la surface du revêtement supérieur (2).

3. Couche selon la revendication 1 ou 2,
caractérisée en ce que
le revêtement inférieur (3) est contrecollé de toute la surface dans les zones intérieures des barrières d'étanchéité (8, 8') en boucle.

4. Couche selon la revendication 1 ou 2,
caractérisée en ce que
le revêtement inférieur (3) est contrecollé dans les zones intérieures des barrières d'étanchéité (8, 8') en boucle par une trace mince d'un adhésif (16) ou par une suite de taches d'un adhésif, qui se trouvent dans le domaine inférieur des barrières d'étanchéité.
